# EUROPEAN PATENT APPLICATION

(11) **EP 4 599 819 A1**
(43) Date of publication of application: **13.08.2025**
(21) Application number: 23874677.0
(22) Date of filing: 22.09.2023
(51) Int. Cl.: A61K 8/39, A61K 8/02, A61K 8/45, A61K 8/63, A61K 8/86, A61Q 1/00, A61Q 5/02, A61Q 5/06, A61Q 17/04, A61Q 19/10

(54) **GEL COMPOSITION AND OIL-IN-WATER-TYPE COMPOSITION**

(30) Priority: 07.10.2022 JP 2022162092
(71) Applicant: Shiseido Company, Ltd., Chuo-ku Tokyo 104-0061 (JP)
(72) Inventor: MIYAHARA Reiji, Tokyo 104-0061 (JP); HIYOSHI Junya, Tokyo 104-0061 (JP); YOSHIMURA Mika, Tokyo 104-0061 (JP)
(74) Representative: Ter Meer Steinmeister & Partner
(86) International application number: PCT/JP2023/034503
(87) International publication number: WO 2024/075548

(57) **Abstract**

A gel composition comprises a first compound represented by polyoxyethylene phytosterol, a second compound represented by polyethylene glycol distearate, a third compound represented by a polyoxyethylene alkyl ether, and a fourth compound that is a two-chain amphiphile having a nitrogen atom. The first compound is present in an amount of 49 to 88% by mass based on a total mass of the first compound, the second compound, and the third compound. The second compound is present in an amount of 6 to 23% by mass, based on the total mass. The third compound is present in an amount of 6 to 28% by mass, based on the total mass. The fourth compound is present in an amount of 0.04 to 0.22 parts by mass, based on 1 part by mass of the total mass.

## Description

### RELATED APPLICATION

This application claims priority from Japanese Patent Application No. 2022-162092 (filed October 7, 2022), the entire disclosure of which is incorporated herein by reference.

### FIELD OF THE INVENTION

The present disclosure relates to a gel composition. The present disclosure also relates to an oil-in-water composition comprising the gel composition.

### BACKGROUND OF THE INVENTION

α-Gel compositions, which can be used as skin external preparations and emulsifiers, have been known (see, for example, Patent Literature 1).

The α-gel composition disclosed in Patent Literature 1 comprises polyoxyethylene (6 mol) distearate, polyoxyethylene (10 mol) behenyl ether, and polyoxyethylene (10 mol) phytosterol.

### [Citation List]

### [Patent Literature]

[Patent Literature 1] Japanese Unexamined Patent Publication No. 2017-132765

### DISCLOSURE OF THE INVENTION

### PROBLEM TO BE SOLVED BY THE INVENTION

The following analysis is provided from the viewpoint of the present disclosure.

The stratum corneum is composed of corneocytes and intercellular lipids present between corneocytes. Intercellular lipids have a lamellar structure composed of ceramides, fatty acids, cholesterol, and the like. Intercellular lipids are believed to have the function of regulating the arrangement of corneocytes, as well as the function of protecting the skin from external stimuli while suppressing moisture loss from the skin (barrier function).

When skin external preparations such as cosmetics contain a component having the barrier function like intercellular lipids, such skin external preparations can exhibit a strong barrier function. For example, since the α-gel composition disclosed in Patent Literature 1 does not contain an intercellular lipid-related component, the composition cannot exhibit a strong barrier function.

Therefore, there is a need for a composition having a strong barrier function.

### MEANS TO SOLVE THE PROBLEM

According to a first aspect of the present disclosure, there is provided a gel composition comprising a first compound represented by the formula shown in the following chemical formula 1; a second compound represented by the formula shown in the following chemical formula 2; a third compound represented by the formula shown in the following chemical formula 3; and a fourth compound that is a two-chain amphiphile having a nitrogen atom. The first compound is present in an amount of 49 to 88% by mass based on a total mass of the first compound, the second compound, and the third compound. The second compound is present in an amount of 6 to 23% by mass, based on the total mass of the first compound, the second compound, and the third compound. The third compound is present in an amount of 6 to 28% by mass, based on the total mass of the first compound, the second compound, and the third compound. The fourth compound is present in an amount of 0.04 to 0.22 parts by mass, based on 1 part by mass of the total mass of the first compound, the second compound, and the third compound. 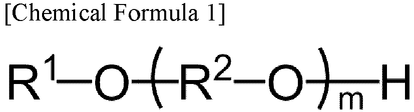
wherein R¹ is at least one selected from the group consisting of a steroid skeleton and a cholesterol skeleton; R² is an alkylene group having 2 to 4 carbon atoms; and m represents an integer of 5 to 30. 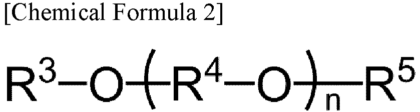
wherein R³ is a straight-chain acyl group or a straight-chain alkyl group having 16 to 24 carbon atoms; R⁴ is an alkylene group having 2 to 4 carbon atoms; R⁵ is a straight-chain acyl group or a straight-chain alkyl group having 16 to 24 carbon atoms; and n represents an integer of 4 to 8. 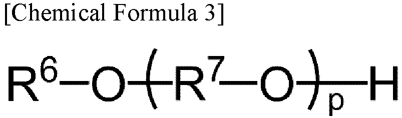
wherein R⁶ is a straight-chain acyl group or a straight-chain alkyl group having 16 to 24 carbon atoms; R⁷ is an alkylene group having 2 to 4 carbon atoms; and p represents an integer of 5 to 30.

According to a second aspect of the present disclosure, there is provided an oil-in-water composition comprising the gel composition according to the first aspect and an oil component. At least a portion of the oil component is incorporated in the gel composition.

### EFFECTS OF THE INVENTION

The gel composition of the present disclosure has a structure similar to that of human intercellular lipids, because of a group of components similar to those of human intercellular lipids. Thus, the gel composition of the present disclosure when applied to the skin can achieve a strong barrier function comparable to that of human intercellular lipids.

The gel composition of the present disclosure can incorporate oil droplets and allows the oil droplets to be stably dispersed in water.

### BRIEF DESCRIPTION OF THE DRAWINGS

[FIG. 1] FIG. 1 shows a small and wide angle X-ray scattering chart in Test Example 1.
[FIG. 2] FIG. 2 shows a small and wide angle X-ray scattering chart in Test Example 2.
[FIG. 3] FIG. 3 shows a small and wide angle X-ray scattering chart in Test Example 3.
[FIG. 4] FIG. 4 shows a small and wide angle X-ray scattering chart in Test Example 4.
[FIG. 5] FIG. 5 shows a differential scanning calorimetry chart in Test Example 4.
[FIG. 6] FIG. 6 shows a polarizing micrograph in Test Example 4.
[FIG. 7] FIG. 7 shows a small and wide angle X-ray scattering chart in Test Example 5.
[FIG. 8] FIG. 8 shows a small and wide angle X-ray scattering chart in Test Example 6.
[FIG. 9] FIG. 9 shows a small and wide angle X-ray scattering chart in Test Example 7.
[FIG. 10] FIG. 10 shows differential scanning calorimetry charts in Test Examples 4 and 8 to 10.
[FIG. 11] FIG. 11 shows a small and wide angle X-ray scattering chart in Test Example 11.
[FIG. 12] FIG. 12 shows a small and wide angle X-ray scattering chart in Test Example 12.
[FIG. 13] FIG. 13 shows a small and wide angle X-ray scattering chart in Test Example 13.
[FIG. 14] FIG. 14 shows a small and wide angle X-ray scattering chart in Test Example 14.
[FIG. 15] FIG. 15 shows a small and wide angle X-ray scattering chart in Test Example 15.
[FIG. 16] FIG. 16 shows a differential scanning calorimetry chart in Test Example 15.
[FIG. 17] FIG. 17 shows a polarizing micrograph in Test Example 15.
[FIG. 18] FIG. 18 shows a small and wide angle X-ray scattering chart in Test Example 16.
[FIG. 19] FIG. 19 shows a differential scanning calorimetry chart in Test Example 16.
[FIG. 20] FIG. 20 shows a polarizing micrograph in Test Example 16.
[FIG. 21] FIG. 21 shows a small and wide angle X-ray scattering chart in Test Example 17.
[FIG. 22] FIG. 22 shows a differential scanning calorimetry chart in Test Example 17.
[FIG. 23] FIG. 23 shows a polarizing micrograph in Test Example 17.
[FIG. 24] FIG. 24 shows a small and wide angle X-ray scattering chart in Test Example 18.
[FIG. 25] FIG. 25 shows a differential scanning calorimetry chart in Test Example 18.
[FIG. 26] FIG. 26 shows a polarizing micrograph in Test Example 18.
[FIG. 27] FIG. 27 shows a polarizing micrograph in Test Example 19.
[FIG. 28] FIG. 28 shows a partially enlarged photograph of the polarizing micrograph in FIG. 27.
[FIG. 29] FIG. 29 is a schematic diagram of an emulsified state in Test Example 19.
[FIG. 30] FIG. 30 is a schematic diagram of a barrier function test apparatus.
[FIG. 31] FIG. 31 shows the results of barrier function tests.
[FIG. 32] FIG. 32 shows the results of barrier function tests.

### BEST MODE FOR CARRYING OUT THE INVENTION

Preferred embodiments of each of the aspects will be hereinafter described.

According to a preferred embodiment of the first aspect, the first compound, the second compound, the third compound, and the fourth compound form a lamellar gel phase.

According to a preferred embodiment of the first aspect, the gel composition further comprises a fifth compound having a steroid skeleton. The fifth compound is present in an amount of 0.04 to 0.22 parts by mass, based on 1 part by mass of the total mass of the first compound, the second compound, and the third compound.

According to a preferred embodiment of the first aspect, the first compound, the second compound, the third compound, the fourth compound, and the fifth compound form a lamellar gel phase.

According to a preferred embodiment of the first aspect, the gel composition further comprises a sixth compound containing at least one selected from the group consisting of higher fatty acids having 14 to 22 carbon atoms. The sixth compound is present in an amount of 0.04 to 0.22 parts by mass, based on 1 part by mass of the total mass of the first compound, the second compound, and the third compound.

According to a preferred embodiment of the first aspect, the first compound, the second compound, the third compound, the fourth compound, and the sixth compound form a lamellar gel phase.

According to a preferred embodiment of the first aspect, the gel composition further comprises a fifth compound having a steroid skeleton; and a sixth compound containing at least one selected from the group consisting of higher fatty acids having 14 to 22 carbon atoms. The fifth compound is present in an amount of 0.04 to 0.22 parts by mass, based on 1 part by mass of the total mass of the first compound, the second compound, and the third compound. The sixth compound is present in an amount of 0.04 to 0.22 parts by mass, based on 1 part by mass of the total mass of the first compound, the second compound, and the third compound.

According to a preferred embodiment of the first aspect, the first compound, the second compound, the third compound, the fourth compound, the fifth compound, and the sixth compound form a lamellar gel phase.

According to a preferred embodiment of the first aspect, the fifth compound is at least one selected from the group consisting of phytosterol, cholesterol, and oryzanol.

According to a preferred embodiment of the first aspect, the total mass of the first compound, the second compound, and the third compound constitutes 29 to 66% by mass of the mass of the gel composition.

According to a preferred embodiment of the first aspect, the fourth compound is at least one selected from the group consisting of ceramide and lecithin.

According to a preferred embodiment of the first aspect, the ceramide is at least one selected from the group consisting of ceramide 1, ceramide 2, ceramide 3, ceramide 3B, ceramide 5, and ceramide 6.

According to a preferred embodiment of the first aspect, the gel composition has a peak indicating a hexagonal structure in an X-ray scattering pattern.

According to a preferred embodiment of the first aspect, the gel composition further comprises water.

According to a preferred embodiment of the second aspect, the oil-in-water composition further comprises water.

According to a preferred embodiment of the second aspect, the oil component is present in an amount of 1 to 50 parts by mass, based on 1 part by mass of the gel composition.

According to a preferred embodiment of the second aspect, the oil component contains at least one selected from the group consisting of phytosteryl/octyldodecyl lauroyl glutamate, polybutylene glycol, castor oil, and phytosteryl macadamiate.

In the following description, PEG is the abbreviation of polyethylene glycol; POE is the abbreviation of polyoxyethylene; POP is the abbreviation of polyoxypropylene; and the number in parentheses after PEG, POE, or POP represents the average molar number of addition of PEG, POE, or POP group(s) in the compound.

As used herein, the term "substantial amount" refers to an amount capable of producing an effect based on the addition of the compound.

### [First Embodiment]

A gel composition according to a first embodiment of the present disclosure will be described. The gel composition of the present disclosure is a composition containing a group of components that are partially or entirely different from the group of components contained in an intercellular lipid present in the skin, and is preferably a composition in which the group of components form a lamellar structure (lamellar gel phase) similar to that of the intercellular lipid. The gel composition of the present disclosure can also be referred to as "pseudo-intercellular lipid". In the gel composition of the present disclosure, the lamellar gel phase more preferably has an α structure (hexagonal structure).

As used herein, the term "lamellar gel phase" refers to a gel-like substance formed by an aggregate of lamellar bilayer membranes formed by the constituents of the gel composition in the presence of water. As used herein, the term "α-gel" refers to a substance with a lamellar gel phase at least partially having an α structure (hexagonal structure). In general, however, a lamellar gel refers to a gel that is an aggregate formed by a higher aliphatic alcohol and a hydrophilic surfactant in water and has an α structure ("Physical Chemistry of Cetyl Alcohol" written by Shoji Fukushima, published by Fragrance Journal).

The formation of a lamellar gel phase can be confirmed by X-ray scattering pattern analysis. For example, when a plurality of peaks corresponding to the long spacing are obtained in the small-angle region, it can be determined that a lamellar gel phase has been formed.

The formation of an α structure (hexagonal structure) can be confirmed by X-ray scattering pattern analysis. For example, when the presence of a peak in the wide-angle region (close to scattering vector q = 1.5 nm⁻¹) can be observed in addition to the presence of the lamellar gel phase described above, it can be determined that an α structure (hexagonal structure) has been formed.

The gel composition according to the first embodiment of the present disclosure comprises a first compound represented by the formula shown in the following chemical formula 4, a second compound represented by the formula shown in the following chemical formula 5, a third compound represented by the formula shown in the following chemical formula 6, and a fourth compound containing at least one selected from the group consisting of ceramide and lecithin.

### [First Compound]

The first compound may be a compound represented by the formula shown in the chemical formula 4. **In** the formula shown in the chemical formula 4, R¹ is at least one selected from the group consisting of a steroid skeleton and a cholesterol skeleton. R¹ may be a moiety other than a hydroxy group, of at least one selected from the group consisting of phytosterol, cholesterol, and ergosterol. R² is an alkylene group having 2 to 4 carbon atoms. m represents an integer of 5 or more, or 10 or more. Furthermore, m represents an integer of 30 or less, or 20 or less.

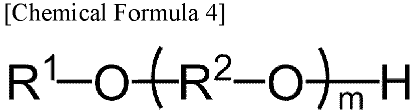

Examples of the first compound include polyoxyethylene (5 mol) phytosterol (e.g., Nikkol BPS-5, manufactured by Nikko Chemicals, Co., Ltd.), polyoxyethylene (10 mol) phytosterol (e.g., Nikkol BPS-10, manufactured by Nikko Chemicals, Co., Ltd.), polyoxyethylene (20 mol) phytosterol (e.g., Nikkol BPS-20, manufactured by Nikko Chemicals, Co., Ltd.), polyoxyethylene (30 mol) phytosterol (e.g., Nikkol BPS-30, manufactured by Nikko Chemicals, Co., Ltd.), and polyoxyethylene (10 mol) cholesterol (e.g., Emalex CS-10, manufactured by Nihon Emulsion Co., Ltd.).

The first compound is present in an amount of preferably 49% by mass or more, more preferably 55% by mass or more, more preferably 60% by mass or more, still more preferably 62% by mass or more, based on the total mass of the first compound, the second compound, and the third compound. The first compound is present in an amount of preferably 88% by mass or less, more preferably 82% by mass or less, still more preferably 76% by mass or less, even more preferably 73% by mass or less, based on the total mass of the first compound, the second compound, and the third compound.

The first compound may be present in an amount of, for example, 2% by mass or more, 5% by mass or more, 10% by mass or more, 15% by mass or more, 20% by mass or more, or 30% by mass or more, based on the mass of the gel composition. The first compound may be present in an amount of, for example, 50% by mass or less, 45% by mass or less, 35% by mass or less, 30% by mass or less, 25% by mass or less, or 20% by mass or less, based on the mass of the gel composition.

### [Second Compound]

The second compound may be a compound represented by the formula shown in the chemical formula 5. In the formula shown in the chemical formula 5, R³ is a straight-chain acyl group or a straight-chain alkyl group having 16 to 24 carbon atoms. R⁴ is an alkylene group having 2 to 4 carbon atoms. R⁵ is a straight-chain acyl group or a straight-chain alkyl group having 16 to 24 carbon atoms. n represents an integer of 4 to 8.

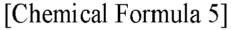

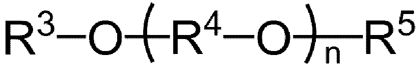

Examples of the second compound include polyoxyethylene (4 mol) distearate (e.g., Emalex 200DIS, manufactured by Nihon Emulsion Co., Ltd.), polyoxyethylene (6 mol) distearate (e.g., Emalex 300DIS, manufactured by Nihon Emulsion Co., Ltd.), polyoxyethylene (8 mol) distearate (e.g., Emalex 400DIS, manufactured by Nihon Emulsion Co., Ltd.), steareth-4 stearate (e.g., Emalex SWS-4, manufactured by Nihon Emulsion Co., Ltd.), steareth-6 stearate (e.g., Emalex SWS-6, manufactured by Nihon Emulsion Co., Ltd.), and polyoxyethylene (8 mol) dibehenyl ether. The binding form of the polyoxyethylene chain and the alkyl group may contain an ester, an ether, or both.

The second compound is present in an amount of preferably 6% by mass or more, more preferably 8% by mass or more, more preferably 10% by mass or more, still more preferably 11% by mass or more, based on the total mass of the first compound, the second compound, and the third compound. The second compound is present in an amount of preferably 23% by mass or less, more preferably 21% by mass or less, still more preferably 19% by mass or less, even more preferably 18% by mass or less, based on the total mass of the first compound, the second compound, and the third compound.

The second compound may be present in an amount of, for example, 0.1% by mass or more, 0.5% by mass or more, 1% by mass or more, 2% by mass or more, 3% by mass or more, or 4% by mass or more, based on the mass of the gel composition. The second compound may be present in an amount of, for example, 20% by mass or less, 16% by mass or less, 12% by mass or less, 10% by mass or less, 8% by mass or less, or 6% by mass or less, based on the mass of the gel composition.

### [Third Compound]

The third compound may be a compound represented by the formula shown in the chemical formula 6. In the formula shown in the chemical formula 6, R⁶ is a straight-chain acyl group or a straight-chain alkyl group having 16 to 24 carbon atoms. R⁷ is an alkylene group having 2 to 4 carbon atoms. p represents an integer of 5 or more, or 10 or more. Furthermore, p represents an integer of 30 or less, or 20 or less.

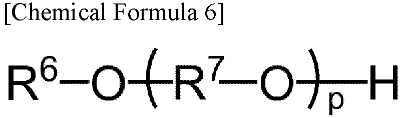

Examples of the third compound include polyoxyethylene (10 mol) behenyl ether (e.g., Nikkol BB-10, manufactured by Nikko Chemicals, Co., Ltd.), polyoxyethylene (20 mol) behenyl ether (e.g., Nikkol BB-20, manufactured by Nikko Chemicals, Co., Ltd.), polyoxyethylene (30 mol) behenyl ether (e.g., Nikkol BB-30, manufactured by Nikko Chemicals, Co., Ltd.), polyoxyethylene (7 mol) cetyl ether (e.g., Emalex 107, manufactured by Nihon Emulsion Co., Ltd.), and polyoxyethylene (10 mol) stearyl ether (e.g., Emalex 610, manufactured by Nihon Emulsion Co, Ltd.).

The third compound is present in an amount of preferably 6% by mass or more, more preferably 8% by mass or more, still more preferably 10% by mass or more, even more preferably 11% by mass or more, based on the total mass of the first compound, the second compound, and the third compound. The third compound is present in an amount of preferably 28% by mass or less, more preferably 26% by mass or less, still more preferably 24% by mass or less, even more preferably 23% by mass or less, based on the total mass of the first compound, the second compound, and the third compound.

The third compound may be present in an amount of, for example, 0.1% by mass or more, 0.5% by mass or more, 1% by mass or more, 2% by mass or more, 3% by mass or more, or 4% by mass or more, based on the mass of the gel composition. The third compound may be present in an amount of, for example, 20% by mass or less, 16% by mass or less, 12% by mass or less, 10% by mass or less, 8% by mass or less, or 6% by mass or less, based on the mass of the gel composition.

The total mass of the first compound, the second compound, and the third compound preferably constitutes 29% by mass or more, more preferably 33% by mass or more, still more preferably 37% by mass or more, even more preferably 41% by mass or more, of the mass of the gel composition. The total mass of the first compound, the second compound, and the third compound preferably constitutes 66% by mass or less, more preferably 61% by mass or less, still more preferably 59% by mass or less, even more preferably 54% by mass or less, of the mass of the gel composition.

### [Fourth Compound]

The fourth compound is a double-chain amphiphile having a nitrogen atom. The term "double-chain" means that the amphiphile has two carbon chains, preferably hydrocarbon chains having 5 to 24 carbon atoms. The hydrocarbon chains may be saturated or unsaturated. The hydrocarbon chains are preferably straight-chain. An amphiphile is a substance having a hydrophilic moiety and a hydrophobic moiety. The fourth compound may contain, for example, at least one selected from the group consisting of ceramide and lecithin.

The ceramide may be a natural ceramide or a synthetic ceramide. The synthetic ceramide may have a structure identical or similar to that of the natural ceramide. The ceramide is preferably capable of forming a lamellar gel structure with at least the first compound, the second compound, and the third compound. The ceramide is preferably a human ceramide. More preferably, the ceramide is at least one selected from the group consisting of ceramide 1, ceramide 2, ceramide 3, ceramide 3B, ceramide 5, and ceramide 6.

The lecithin may be, for example, at least one selected from the group consisting of soybean lecithin, egg yolk lecithin, and hydrogenated lecithin. The lecithin may also be phosphatidylcholine.

The fourth compound is present in an amount of preferably 0.04 parts by mass or more, more preferably 0.06 parts by mass or more, still more preferably 0.08 parts by mass or more, based on 1 part by mass of the total amount (total mass) of the first compound, the second compound, and the third compound in the gel composition. The fourth compound is present in an amount of preferably 0.22 parts by mass or less, more preferably 0.20 parts by mass or less, still more preferably 0.17 parts by mass or less, even more preferably 0.14 parts by mass or less, based on 1 part by mass of the total amount of the first compound, the second compound, and the third compound in the gel composition.

The fourth compound may be present in an amount of, for example, 0.1% by mass or more, 0.5% by mass or more, 1% by mass or more, 2% by mass or more, 3% by mass or more, or 4% by mass or more, based on the mass of the gel composition. The fourth compound may be present in an amount of, for example, 20% by mass or less, 16% by mass or less, 12% by mass or less, 10% by mass or less, 8% by mass or less, or 6% by mass or less, based on the mass of the gel composition.

When the gel composition does not contain the fifth compound and the sixth compound described below, it is preferred that the first compound, the second compound, the third compound, and the fourth compound form a lamellar gel phase in the gel composition.

The gel composition may further comprise water. The water may be present in an amount of, for example, 10% by mass or more, 15% by mass or more, 20% by mass or more, 25% by mass or more, 30% by mass or more, or 35% by mass or more, based on the mass of the gel composition. The water may be present in an amount of, for example, 60% by mass or less, 55% by mass or less, 50% by mass or less, 45% by mass or less, 40% by mass or less, or 35% by mass or less, based on the mass of the gel composition.

According to the gel composition of the first embodiment, the gel composition when applied to the skin can exhibit a skin barrier function. For example, moisture loss from the inside of the skin can be suppressed (occlusion).

The gel composition according to the first embodiment has a high barrier function.

The gel composition according to the first embodiment incorporates oil droplets and allows the oil droplets to be stably dispersed in water.

### [Second Embodiment]

A gel composition according to a second embodiment of the present disclosure will be described. The gel composition according to the second embodiment of the present disclosure further comprises a fifth compound, in addition to the components in the gel composition according to the first embodiment.

### [Fifth Compound]

The fifth compound is a compound having a steroid skeleton (cyclopentanohydrophenanthrene ring). The fifth compound may be, for example, at least one selected from the group consisting of phytosterol, cholesterol, and oryzanol (y-oryzanol).

When the gel composition does not contain the sixth compound, it is preferred that the first compound, the second compound, the third compound, the fourth compound, and the fifth compound form a lamellar gel phase in the gel composition.

The fifth compound is present in an amount of preferably 0.04 parts by mass or more, more preferably 0.06 parts by mass or more, still more preferably 0.08 parts by mass or more, based on 1 part by mass of the total mass of the first compound, the second compound, and the third compound in the gel composition. The fifth compound is present in an amount of preferably 0.22 parts by mass or less, more preferably 0.20 parts by mass or less, still more preferably 0.17 parts by mass or less, even more preferably 0.14 parts by mass or less, based on 1 part by mass of the total mass of the first compound, the second compound, and the third compound in the gel composition.

The fifth compound may be present in an amount of, for example, 0.1% by mass or more, 0.5% by mass or more, 1% by mass or more, 2% by mass or more, 3% by mass or more, or 4% by mass or more, based on the mass of the gel composition. The fifth compound may be present in an amount of, for example, 20% by mass or less, 16% by mass or less, 12% by mass or less, 10% by mass or less, 8% by mass or less, or 6% by mass or less, based on the mass of the gel composition.

For components other than the fifth compound, reference is made to the description in the first embodiment.

The gel composition according to the second embodiment can produce the same effect as that of the first embodiment. According to the second embodiment, the stability of the gel composition can be improved more than in the first embodiment.

### [Third Embodiment]

A gel composition according to a third embodiment of the present disclosure will be described. The gel composition according to the third embodiment of the present disclosure further comprises a sixth compound, in addition to the components in the gel composition according to the first embodiment.

### [Sixth Compound]

The sixth compound may be at least one selected from the group consisting of higher fatty acids having 14 to 22 carbon atoms. Examples of the sixth compound include stearic acid.

When the gel composition does not contain the fifth compound, it is preferred that the first compound, the second compound, the third compound, the fourth compound, and the sixth compound form a lamellar gel phase in the gel composition.

The sixth compound is present in an amount of preferably 0.04 parts by mass or more, more preferably 0.06 parts by mass or more, still more preferably 0.08 parts by mass or more, based on 1 part by mass of the total mass of the first compound, the second compound, and the third compound in the gel composition. The sixth compound is present in an amount of preferably 0.22 parts by mass or less, more preferably 0.20 parts by mass or less, still more preferably 0.17 parts by mass or less, even more preferably 0.14 parts by mass or less, based on 1 part by mass of the total mass of the first compound, the second compound, and the third compound in the gel composition.

The sixth compound may be present in an amount of, for example, 0.1% by mass or more, 0.5% by mass or more, 1% by mass or more, 2% by mass or more, 3% by mass or more, or 4% by mass or more, based on the mass of the gel composition. The sixth compound may be present in an amount of, for example, 20% by mass or less, 16% by mass or less, 12% by mass or less, 10% by mass or less, 8% by mass or less, or 6% by mass or less, based on the mass of the gel composition.

For components other than the sixth compound, reference is made to the description in the first embodiment.

The gel composition according to the third embodiment can produce the same effect as that of the first embodiment. According to the third embodiment, the stability of the gel composition can be improved more than in the first embodiment.

### [Fourth Embodiment]

A gel composition according to a fourth embodiment of the present disclosure will be described. The gel composition according to the fourth embodiment of the present disclosure further comprises a fifth compound and a sixth compound, in addition to the components in the gel composition according to the first embodiment.

For the fifth compound, reference is made to the description in the second embodiment. For the sixth compound, reference is made to the description in the third embodiment. For components other than the fifth and sixth compounds, reference is made to the description in the first embodiment.

It is preferred that the first compound, the second compound, the third compound, the fourth compound, the fifth compound, and the sixth compound form a lamellar gel phase in the gel composition.

The gel composition according to the fourth embodiment can produce the same effect as that of the first embodiment. According to the fourth embodiment, the stability of the gel composition can be improved more than in the first to third embodiments.

### [Fifth Embodiment]

An oil-in-water composition according to a fifth embodiment of the present disclosure will be described. The oil-in-water composition according to the fifth embodiment of the present disclosure comprises the gel composition according to at least one of the first to fourth embodiments and an oil component. The oil component in the fifth embodiment excludes the first to sixth compounds described above.

At least a portion of the oil component is incorporated in the gel composition. The oil component can exist as oil droplets and be in an emulsified state by the gel composition.

The oil component may have an average particle diameter of, for example, 0.5 µm or more. The oil component may have an average particle diameter of, for example, 10 µm or less.

The oil component is not specifically limited, and, for example, liquid oils and fats, solid oils and fats, waxes, hydrocarbon oils, higher fatty acids, higher alcohols, synthetic ester oils, and silicone oils may be suitably blended as the oil component. The oil component is preferably in liquid form at 25°C and at atmospheric pressure.

The oil component preferably contains a component for softening the stratum corneum (stratum corneum softening component). Examples of the stratum corneum softening component include at least one selected from the group consisting of phytosteryl/octyldodecyl lauroyl glutamate, polybutylene glycol, castor oil, and phytosteryl macadamiate.

Examples of liquid oils and fats include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, Chinese tung oil, Japanese tung oil, jojoba oil, germ oil, and triglycerin.

Examples of solid oils and fats include cacao butter, coconut oil, horse fat, hydrogenated coconut oil, palm oil, beef tallow, mutton tallow, hydrogenated beef tallow, palm kernel oil, lard, beef bone tallow, Japan wax kernel oil, hardened oil, neat's foot oil, Japan wax, and hydrogenated castor oil.

Examples of waxes include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of hydrocarbon oils include liquid paraffin, ozocerite, squalene, pristane, paraffin, ceresin, squalane, petrolatum, and microcrystalline wax.

Examples of higher fatty acids include lauric acid, myristic acid, palmitic acid, stearic acid, behenic acid, oleic acid, undecylenic acid, tall oil acid, isostearic acid, linoleic acid, linolenic acid, eicosapentaenoic acid (EPA), and docosahexaenoic acid (DHA).

Examples of higher alcohols that can be used include straight-chain alcohols (e.g., lauryl alcohol, cetyl alcohol, stearyl alcohol, behenyl alcohol, myristyl alcohol, oleyl alcohol, and cetostearyl alcohol); and branched-chain alcohols (e.g., monostearyl glycerin ether (batyl alcohol), 2-decyltetradecinol, lanolin alcohol, cholesterol, phytosterol, hexyl dodecanol, isostearyl alcohol, and octyl dodecanol).

Examples of synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, tri-2-heptylundecanoic acid glyceride, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of silicone oils include silicone compounds such as dimethylpolysiloxane, methylhydrogenpolysiloxane, methylphenylpolysiloxane, stearoxymethylpolysiloxane, polyether-modified organopolysiloxane, fluoroalkyl-polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicate, silicone RTV rubber, and cyclopentasiloxane.

The stratum corneum softening component is preferably contained in an amount of 0.5% by mass or more, based on the mass of the oil-in-water composition. The stratum corneum softening component may be present in an amount of, for example, 1% by mass or more, 2% by mass or more, or 3% by mass or more, based on the mass of the oil-in-water composition. The stratum corneum softening component may be present in an amount of, for example, 15% by mass or less, 12% by mass or less, 10% by mass or less, 8% by mass or less, or 5% by mass or less, based on the mass of the oil-in-water composition.

The oil component may contain an oil component having a molecular weight of 400 or less. The inclusion of an oil component having a molecular weight of 400 or less can improve the cleansing ability for oily matter to be cleansed off, such as makeup. Examples of the oil component having a molecular weight of 400 or less include at least one selected from the group consisting of, for example, tripropylene glycol dipivalate, isodecane, isononyl isononanoate, cetyl ethylhexanoate, ethyl isostearate, isobutyl isostearate, isodecyl neopentanoate, octyldodecyl neopentanoate, myristyl neopentanoate, and isostearyl neopentanoate.

The oil component having a molecular weight of 400 or less may be present in an amount of, for example, 50% by mass or more, 60% by mass or more, 70% by mass or more, 80% by mass or more, 90% by mass or more, or 100% by mass, based on the mass of the oil component.

The oil component is present in an amount of preferably 1 part by mass or more, based on 1 part by mass of the total mass of the first compound, the second compound, the third compound, the fourth compound, the fifth compound, and the sixth compound (that is, the mass of the gel composition excluding water). The oil component may be present in an amount of, for example, 5 parts by mass or more, 10 parts by mass or more, 15 parts by mass or more, 20 parts by mass or more, 25 parts by mass or more, 30 parts by mass or more, 35 parts by mass or more, or 40 parts by mass or more, based on 1 part by mass of the total mass of the first to sixth compounds. If the amount of the oil component is less than 1 part by mass, the effect of the oil component cannot be sufficiently achieved. The oil component is preferably present in an amount of 50 parts by mass or less, based on 1 part by mass of the total mass of the first to sixth compounds. The oil component may be present in an amount of, for example, 45 parts by mass or less, 40 parts by mass or less, 35 parts by mass or less, 30 parts by mass or less, 25 parts by mass or less, 20 parts by mass or less, 15 parts by mass or less, or 10 parts by mass or less, based on 1 part by mass of the total mass of the first to sixth compounds. If the amount of the oil component exceeds 50 parts by mass, the oil component is difficult to be incorporated in the gel composition.

The content of the oil component may be, for example, 1% by mass or more or 5% by mass or more, based on the mass of the oil-in-water composition. The content of the oil component may be, for example, 50% by mass or less or 25% by mass or less, based on the mass of the oil-in-water composition.

The oil-in-water composition of the present disclosure may further comprise water other than the water contained in the gel composition. The water content may be 10% by mass or more, 15% by mass or more, 20% by mass or more, 25% by mass or more, 30% by mass or more, 35% by mass or more, or 40% by mass or more, based on the mass of the composition. The water content may be 90% by mass or less, 80% by mass or less, 70% by mass or less, 60% by mass or less, 50% by mass or less, 40% by mass or less, 30% by mass or less, 25% by mass or less, or 20% by mass or less, based on the mass of the composition. As used herein, the water content includes not only water in the aqueous phase but also water contained in the gel composition.

The oil-in-water composition of the present disclosure may further comprise a powder. The powder may be a powder having a hydrophobic particle surface. The hydrophobic powder may include a powder having a particle surface subjected to a hydrophobic treatment. It is believed that at least a portion of the hydrophobic powder is contained in the oil droplet particles (oil phase) of the oil-in-water composition.

A portion of the hydrophobic powder may be present in the aqueous phase. It is believed that the gel composition of the present disclosure adheres to at least a portion of the surface of the hydrophobic powder in the aqueous phase. This results in improved dispersibility of the hydrophobic powder even in the aqueous phase.

Examples of methods of the hydrophobic treatment for the powder include subjecting the powder to a hydrophobic treatment by a wet process using a solvent, a vapor phase process, a mechanochemical process, or the like, using a silicone resin such as methyl hydrogen polysiloxane, dimethylpolysiloxane, a dextrin fatty acid ester, a higher fatty acid, a higher alcohol, a fatty acid ester, a metal soap, an alkyl phosphate ether, a fluorine compound, or a hydrocarbon such as squalane or paraffin. The hydrophobic powder may be, for example, a powder of a metal oxide that has been subjected to the hydrophobic treatment. The metal oxide may be, for example, a powder that acts as an ultraviolet scattering agent. Examples of the metal oxide include zinc oxide, titanium oxide, iron oxide, and cerium oxide.

Primary particles of the hydrophobic powder may have an average particle diameter of 10 nm or more. The primary particles of the hydrophobic powder may have an average particle diameter of, for example, 500 nm or less, 200 nm or less, 100 nm or less, 50 nm or less, or 40 nm or less. The average particle diameter of the primary particles of the hydrophobic powder can be measured using the dynamic light scattering method.

When a plurality of types of hydrophobic powders are present, the average particle diameter of the primary particles of the hydrophobic powders can be calculated in consideration of the ratio of the powders present. That is, the sum of values obtained by multiplying the particle diameter (catalog value) of each powder by the proportion by mass of the powder to the total amount of the hydrophobic powders may be adopted.

The powder is not specifically limited as long as the powder can be used for general purposes such as cosmetics. Examples of powders that can be used include inorganic powders (e.g., talc, kaolin, mica, sericite, muscovite, phlogopite, synthetic mica, lepidolite, biotite, lithia mica, calcined mica, calcined talc, vermiculite, magnesium carbonate, calcium carbonate, aluminum silicate, barium silicate, calcium silicate, magnesium silicate, strontium silicate, metal tungstates, magnesium, silica, zeolite, glass, barium sulfate, calcined calcium sulfate (calcined gypsum), calcium phosphate, fluorine apatite, hydroxyapatite, ceramic powder, metal soaps (e.g., zinc myristate, calcium palmitate, and aluminum stearate), and boron nitride); organic powders (e.g., polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, cellulose powder, silicone resin powder, silk powder, wool powder, and urethane powder); inorganic white pigments (e.g., titanium dioxide and zinc oxide); inorganic red-type pigments (e.g., iron oxide (bengara) and iron titanate); inorganic brown-type pigments (e.g., γ-iron oxide), inorganic yellow-type pigments (e.g., yellow iron oxide and yellow ocher), inorganic black-type pigments (e.g., black iron oxide, carbon black, and lower titanium oxide), inorganic violet-type pigments (e.g., manganese violet and cobalt violet); inorganic green-type pigments (e.g., chromium oxide, chromium hydroxide, and cobalt titanate); inorganic blue-type pigments (e.g., ultramarine and prussian blue); pearl pigments (e.g., titanium oxide coated mica, titanium oxide coated bismuth oxychloride, titanium oxide coated talc, colored titanium oxide coated mica, bismuth oxychloride, and fish scale flakes); metal powder pigments (e.g., aluminum powder and copper powder); organic pigments such as zirconium, barium, or aluminum lake (e.g., organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); natural pigments (e.g., chlorophyll and β-carotene); wax powders (e.g., carnauba wax powder); and starch powders (e.g. corn starch powder and rice starch powder).

The hydrophobic powder is preferably present in an amount of 0.5% by mass or more, based on the mass of the oil-in-water composition. The hydrophobic powder may be present in an amount of, for example, 1% by mass or more, 3% by mass or more, 5% by mass or more, 8% by mass or more, 10% by mass or more, 15% by mass or more, or 20% by mass or more, based on the mass of the oil-in-water composition. If the amount of the hydrophobic powder is less than 0.5% by mass, the effect of the hydrophobic powder cannot be achieved. The hydrophobic powder is preferably present in an amount of 30% by mass or less, based on the mass of the oil-in-water composition. The hydrophobic powder may be present in an amount of, for example, 25% by mass or less, 20% by mass or less, 15% by mass or less, 10% by mass or less, 8% by mass or less, 5% by mass or less, or 3% by mass or less, based on the mass of the oil-in-water composition. If the amount of the hydrophobic powder exceeds 30% by mass, the hydrophobic powder cannot be contained in the oil phase.

The oil-in-water composition of the present disclosure allows the oil component to be stably dispersed. Furthermore, the oil-in-water composition of the present disclosure when applied to the skin can exhibit a high barrier function based on the gel composition of the present disclosure.

In the gel composition and the oil-in-water composition of the present disclosure, it may be impossible or almost impractical to directly specify the phase structure and the like in terms of composition. In such cases, it should be permitted to specify the gel composition and the oil-in-water composition of the present disclosure in terms of production method.

### [Production Method]

A method for producing the gel compositions according to the first to fourth embodiments of the present disclosure will be described. The method for producing the gel compositions may comprise, for example, the step of heating and melting the first to sixth compounds (at least the first to fourth compounds) described above; and the step of mixing the molten mixture with water and stirring the mixture. The first to sixth compounds may be melted, for example, at 70 to 110°C. The water is preferably heated to about the same temperature as that of the mixture (e.g., 70 to 80°C; e.g., ±15°C). The lamellar gel phase can be obtained by cooling the mixture after mixing with water.

A method for producing the oil-in-water composition according to the fifth embodiment of the present disclosure will be described. In a first aspect, the method for producing the oil-in-water composition may comprise, for example, the step of emulsifying an oil component in the gel composition (lamellar gel phase); and the step of adding an aqueous component after emulsification. The step of emulsifying an oil component may include, for example, the step of preparing a solution of the first to sixth compounds (at least the first to fourth compounds) in a polyhydric alcohol (e.g., dipropylene glycol or 1,3-butylene glycol); and the step of emulsifying the oil component while adding the oil component to the solution. For example, the oil-in-water composition of the present disclosure can be produced using a non-aqueous emulsification method (D-phase emulsification method). The emulsified particles can be made finer by using the non-aqueous emulsification method. Furthermore, the lamellar gel phase can be adsorbed onto the oil-water interface.

The oil-in-water composition of the present disclosure may optionally contain other components as appropriate, as long as the effect of the present disclosure is not impaired. Examples of other components include amphoteric surfactants, hydrophilic nonionic surfactants, lipophilic nonionic surfactants, water-soluble polymers, thickeners, moisturizers, film-forming agents, oil-soluble ultraviolet absorbers, water-soluble ultraviolet absorbers, sequestering agents, amino acids, organic amines, polymer emulsions, pH adjusters, skin nutrients, vitamins, antioxidants, antioxidant aids, and fragrances.

Examples of amphoteric surfactants include imidazoline-based amphoteric surfactants (e.g., 2-undecyl-N,N,N-(hydroxyethylcarboxymethyl)-2-imidazoline sodium salt, 2-cocoyl-2-imidazolinium hydroxide-1-carboxyethyloxy)-2-sodium salt); and betaine-based surfactants (e.g., 2-heptadecyl-N-carboxymethyl-N-hydroxyethylimidazolinium betaine, lauryl dimethyl amino acetate betaine, alkyl betaines, amidobetaines, and sulfobetaines).

Examples of hydrophilic nonionic surfactants include POE-sorbitan fatty acid esters (e.g., POE-sorbitan monooleate, POE-sorbitan monostearate, POE-sorbitan monooleate, and POE-sorbitan tetraoleate); POE-sorbit fatty acid esters (e.g., POE-sorbit monolaurate, POE-sorbit monooleate, POE-sorbit pentaoleate, and POE-sorbit monostearate); POE-glycerin fatty acid esters (e.g., POE-monooleates such as POE-glycerin monostearate, POE-glycerin monoisostearate, and POE-glycerin triisostearate); POE-fatty acid esters (e.g., POE-distearate, POE-monodioleate, and ethyleneglycol distearate); POE-alkyl ethers (e.g., POE-lauryl ether, POE-oleyl ether, POE-stearyl ether, POE-behenyl ether, POE-2-octyldodecyl ether, and POE-cholestanol ether); pluronic types (e.g., Pluronic (registered trademark)); POE/POP-alkyl ethers (e.g., POE/POP-cetyl ether, POE/POP-2-decyltetradecyl ether, POE/POP-monobutyl ether, POE/POP-hydrogenated lanoline, and POE/POP glycerin ether); tetra POE/tetra POP-ethylenediamine condensation products (e.g., Tetronic); POE-castor oil hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated oil maleate); POE-beeswax/lanoline derivatives (e.g., POE-sorbit beeswax); alkanolamides (e.g., coconut oil fatty acid diethanolamide, lauric acid monoethanolamide, and fatty acid isopropanolamide); POE-propyleneglycol fatty acid esters; POE-alkyl amines; POE-fatty acid amides; sucrose fatty acid esters; alkylethoxydimethylamine oxide; and trioleyl phosphate.

Examples of lipophilic nonionic surfactants include sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerin polyglycerin fatty acids (e.g., monocottonseed oil fatty acid glycerin, glycerin monoerucate, glycerin sesquioleate, glycerin monostearate, glycerin α,α'-oleate pyroglutamate, and glycerin monostearate maleate); propylene glycol fatty acid esters (e.g., propylene glycol monostearate); hydrogenated castor oil derivatives; and glycerin alkyl ethers.

Examples of natural water-soluble polymers include plant-based polymers (e.g., gum arabic, tragacanth gum, galactan, guar gum, carob gum, karaya gum, carrageenan, pectine, agar, quince seed (marmelo), algae colloid (brown algae extract), starch (rice, corn, potato, and wheat), and glicyrrhizic acid); microorganism-based polymers (e.g., xanthan gum, dextran, succinoglycan, and pullulan); and animal-based polymers (e.g., collagen, casein, albumin, and gelatin).

Examples of semi-synthetic water-soluble polymers include starch-based polymers (e.g., carboxymethyl starch and methylhydroxypropyl starch); cellulose-based polymers (methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, microcrystalline cellulose, and cellulose powder); and alginic acid-based polymers (e.g., sodium alginate and propylene glycol alginate).

Examples of synthetic water-soluble polymers include vinyl-based polymers (e.g., polyvinyl alcohol, polyvinyl methyl ether, polyvinylpyrrolidone, and carboxyvinyl polymer); polyoxyethylene-based polymers (e.g., polyoxyethylene-polyoxypropylene copolymers of polyethylene glycol 20,000, 40,000, or 60,000); acrylic polymers (e.g., sodium polyacrylate, polyethylacrylate, and polyacrylamide); polyethyleneimine; and cationic polymers.

Examples of thickeners include gum arabic, carrageenan, karaya gum, tragacanth gum, carob gum, quince seed (marmelo), casein, dextrin, gelatin, sodium pectinate, sodium alginate, methyl cellulose, ethyl cellulose, carboxymethylcellulose (CMC), hydroxyethylcellulose, hydroxypropylcellulose, polyvinyl alcohol (PVA), polyvinyl methyl ether (PVM), PVP (polyvinylpyrrolidone), sodium polyacrylate, carboxyvinyl polymer, locust bean gum, guar gum, tamarind gum, dialkyldimethylammonium cellulose sulfate, xanthan gum, magnesium aluminum silicate, bentonite, hectolite, magnesium aluminum silicate (veegum), laponite, silicic anhydride, taurate-based synthetic polymers, and acrylate-based synthetic polymers.

Examples of moisturizers include polyethylene glycol, propylene glycol, glycerin, 1,3-butylene glycol, xylitol, sorbitol, maltitol, chondroitin sulfate, hyaluronic acid, mucoitin sulfate, caronic acid, atelocollagen, cholesteryl-12-hydroxystearate, sodium lactate, bile salt, dl-pyrrolidone carboxylic acid salt, alkylene oxide derivatives, short-chain soluble collagen, diglycerin (EO) PO adduct, *rosa roxburghii* fruit extract, *achillea millefolium* extract, and *melilotus officinalis* extract.

Examples of film-forming agents include anionic film-forming agents (e.g., (meth)acrylic acid/(meth)acrylic acid ester copolymer and methyl vinyl ether/maleic anhydride high polymer), cationic film-forming agents (e.g., cationized cellulose, dimethyldiallylammonium chloride polymer, and dimethyldiallylammonium chloride/acrylamide copolymer), and nonionic film-forming agents (e.g., polyvinyl alcohol, polyvinylpyrrolidone, polyvinyl acetate, polyacrylic acid ester copolymer, (meth)acrylamide, polymeric silicone, silicone resin, and trimethylsiloxysilicic acid).

Examples of oil-soluble ultraviolet absorbers include benzoic acid-based ultraviolet absorbers (e.g., p-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, and hexyl diethylaminohydroxybenzoylbenzoate); anthranilic acid-based ultraviolet absorbers (e.g., homomentyl-N-acetyl anthranilate); salicylic acid-based ultraviolet absorbers (e.g., ethylhexyl salicylate, amyl salicylate, menthyl salicylate, homomenthyl salicylate, octyl salicylate, phenyl salicylate, benzyl salicylate, p-isopropanol phenyl salicylate, and homosalicylate); cinnamic acid-based ultraviolet absorbers (e.g., octyl methoxycinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, ethylhexyl methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-di-para-methoxycinnamate); 3-(4'-methylbenzylidene)-d, l-camphor; 3-benzylidene-d, l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenyl acrylate (octocrylene); 2,4-bis-{[4-(2-ethylhexyloxy)-2-hydroxy]-phenyl}-6-(4-methoxyphenyl)-(1,3,5)-triazine; and benzophenone-based ultraviolet absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone).

Examples of water-soluble ultraviolet absorbers include benzophenone-based ultraviolet absorbers (e.g., 2-hydroxy-4-methoxybenzophenone-5-sulfonate), benzylidene camphor-based ultraviolet absorbers (e.g., benzylidene camphor sulfonic acid and terephthalylidene dicamphor sulfonic acid), and phenylbenzimidazole-based ultraviolet absorbers (e.g., phenylbenzimidazole sulfonic acid).

Examples of sequestering agents include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of amino acids include neutral amino acids (e.g., threonine and cysteine) and basic amino acids (e.g., hydroxylysine). Examples of amino acid derivatives include sodium acyl sarcosinate (sodium lauroyl sarcosinate), acyl glutamate, sodium acyl β-alaninate, glutathione, and pyrrolidone carboxylate.

Examples of organic amines include monoethanolamine, diethanolamine, triethanolamine, morpholine, triisopropanol amine, 2-amino-2-methyl-1,3-propanediol, and 2-amino-2-methyl-1-propanol.

Examples of polymer emulsions include acrylic resin emulsion, ethyl polyacrylate emulsion, acrylic resin liquid, polyacrylalkyl ester emulsion, polyvinyl acetate resin emulsion, and natural rubber latex.

Examples of pH adjusters include buffers such as lactic acid-sodium lactate, citric acid-sodium citrate, and succinic acid-sodium succinate.

Examples of vitamins include vitamins A, B1, B2, B6, C, E, and derivatives thereof, pantothenic acid and derivatives thereof, and biotin.

Examples of antioxidants include tocopherols, dibutylhydroxytoluene, butylhydroxyanisol, and gallic acid esters.

Examples of antioxidant aids include phosphoric acid, citric acid, ascorbic acid, maleic acid, malonic acid, succinic acid, fumaric acid, cephalin, hexamethaphosphate, phytic acid, and ethylene diamine tetraacetic acid.

Examples of other components that can be blended include antiseptic agents (ethyl paraben, butyl paraben, chlorphenesin, phenoxyethanol, and the like); antiphlogistic agents (e.g., glycyrrhizic acid derivatives, glycyrrhetinic acid derivatives, salicylic acid derivatives, hinokitiol, zinc oxide, and allantoin); whitening agents (e.g., placenta extract, *saxifraga sarmentosa* extract, and arbutin); various extracts (e.g., *phellodendron amurense* bark, *coptis japonica, lithospermum erythrorhizon* root, *paeonia lactiflora, swertia japonica,* birch, sage, *eriobotrya japonica,* carrot, aloe, mallow, iris, grape, coix seed, *luffa cylindrica,* lily, saffron, *cnidium officinale* root, *zingiber officinale, hypericum erectum, ononis spinosa,* garlic, red pepper, *citrus unshiu, angelica acutiloba,* and seaweed); activating agents (e.g., royal jelly, photosensitizers, and cholesterol derivatives); blood circulation promotion agents (e.g., vanillylamide nonylate, benzyl nicotinate, β-butoxyethyl nicotinate, capsaicine, zingerone, cantharides tincture, ichthammol, tannic acid, α-borneol, tocopherol nicotinate, inositol hexanicotinate, cyclandelate, cinnarizine, tolazoline, acetylcholine, verapamil, cephalantin, and γ-oryzanol); anti-seborrheric agents (e.g., sulfur and thianthol); and anti-inflammatory agents (e.g., tranexamic acid, thiotaurine, and hypotaurine).

### EXAMPLES

The gel composition and the oil-in-water composition of the present disclosure will be hereinafter described with reference to examples. However, the gel composition and the oil-in-water composition of the present disclosure are not limited to the following examples. The content of each component shown in each table is in % by mass.

### [Test Examples 1 to 4]

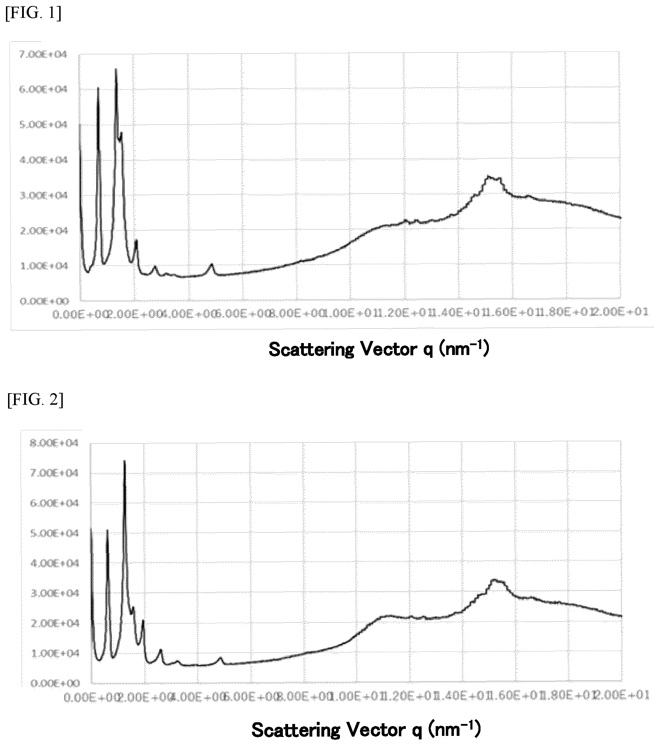

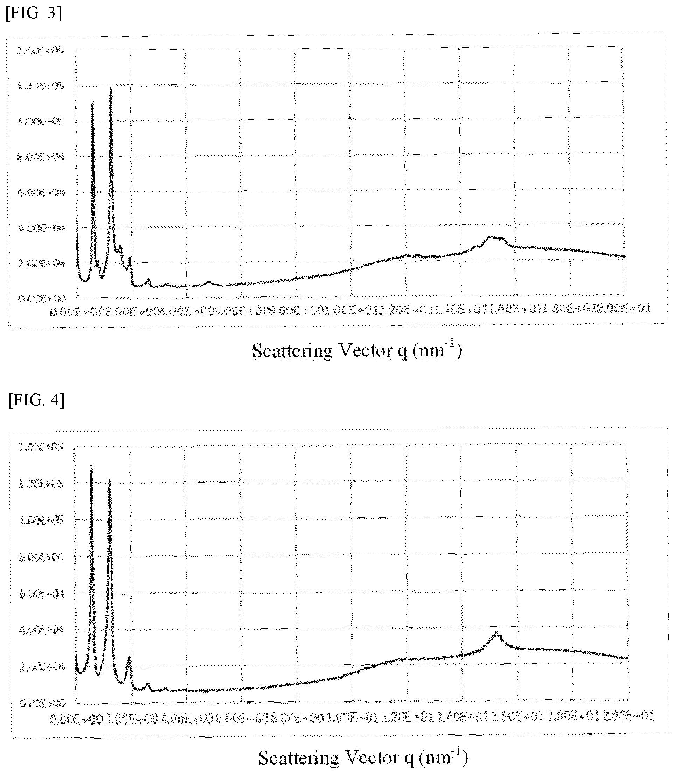

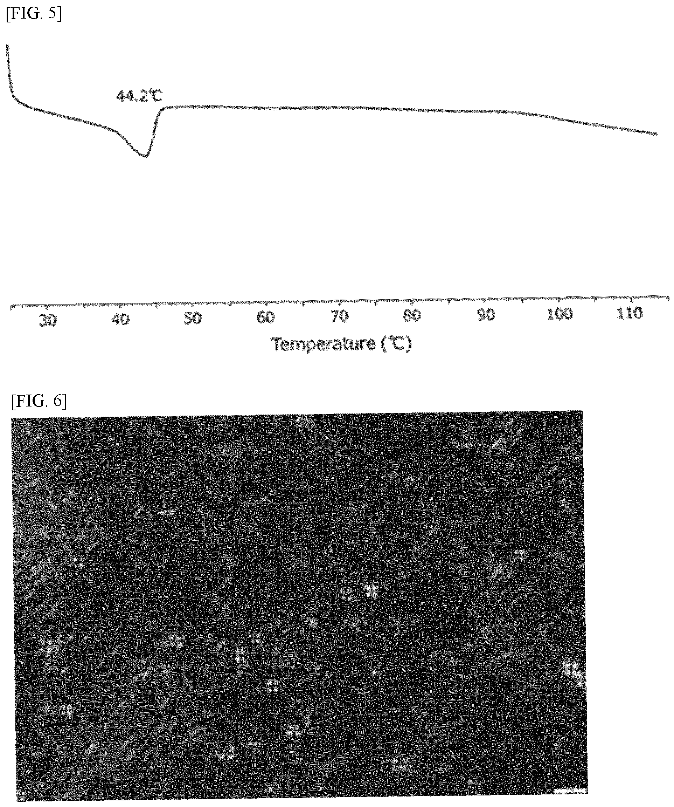

The components shown in Table 1 other than ion-exchanged water were melted at 100°C to give a homogeneous mixture. Ion-exchanged water at 70 to 80°C was added to the mixture, mixed and then cooled. The mixture was then degassed by centrifugation to prepare a gel. The gel of each test example was subjected to small and wide angle X-ray scattering measurement and differential scanning calorimetry measurement (DSC measurement). The gel composition of Test Example 4 was observed with a polarizing microscope. Figures 1 to 4 show small and wide angle X-ray scattering charts of the compositions of Test Examples 1 to 4. FIG. 5 shows a differential scanning calorimetry chart of the composition of Test Example 4. FIG. 6 shows a polarizing microscope image.

### [Small and Wide Angle X-Ray Scattering Measurement]

The composition of each test example was subjected to small and wide angle X-ray scattering measurement using the SWAXS measuring apparatus SAXSees mc2 (manufactured by Anton Paal GmbH, Graz, Austria) at 20°C.

### [Differential Scanning Calorimetry Measurement]

Using a model DSC 1 differential scanning calorimeter (manufactured by Mettler Toledo, Greifense, Switzerland), about 7 mg of the composition of each test example was placed in an aluminum pan, and subjected to measurement by heating to 25 to 115°C at 2°C per minute.

### [Polarizing Microscope Image Taking]

A polarizing microscope image was taken using the model BX53 system polarizing microscope manufactured by Olympus Corporation at a magnification of 400 times, with polarizing plates in crossed Nicols.

In all of X-ray charts in Test Examples 1 to 4, peaks corresponding to the long spacing of a lamellar gel are present on the small-angle side, which indicates that the compositions of Test Examples 1 to 4 have a lamellar structure. The presence of a peak close to scattering vector q = 1.5 nm⁻¹ also indicates that the hexagonal lattice of an α structure exists, and sublattice planes are highly ordered. This confirmed that the obtained compositions were α-gels. The compositions having an α-gel structure can suppress deposition of hydrated crystals and exhibit a high barrier function.

Based on the similarity between the constituents of human intercellular lipids and the constituents in Test Examples 1 to 4, it is believed that the compositions of Test Examples 1 to 4 are similar to human intercellular lipids in components and structure, thus constituting pseudo-intercellular lipids.

The composition of Test Example 4 does not show the presence of a peak derived from ceramide 3. It is therefore believed that the stability of the gel composition can be improved by adding a sterol-based compound and a higher fatty acid.

According to the differential scanning calorimetry chart shown in FIG. 5, the presence of an endothermic peak indicates that at least the composition of Test Example 4 has a eutectic point. When the composition has a eutectic point, the composition can suppress the deposition of a portion of the crystals.

The polarization microscope image shown in FIG. 6 also confirmed the formation of a lamellar gel.

**[Table 1]**

| Test Example | 1 | 2 | 3 | 4 |
|---|---|---|---|---|
| PEG-10 Phytosterol^{*1} | 35.8 | 30.8 | 35.8 | 30.8 |
| PEG-6 Distearate^{*2} | 7.1 | 7.1 | 7.1 | 7.1 |
| Beheneth-10^{*3} | 12.1 | 12.1 | 7.1 | 7.1 |
| Ceramide 3 | 5 | 5 | 5 | 5 |
| Phytosterol | - | 5 | - | 5 |
| Stearic Acid | - | - | 5 | 5 |
| Ion-Exchanged Water | 40 | 40 | 40 | 40 |
| Total | 100 | 100 | 100 | 100 |
| *1 : Nikkol BPS-10 manufactured by Nikko Chemicals Co., Ltd.) | | | | |
| *2 : Emalex 300DIS manufactured by Nihon Emulsion Co., Ltd.) | | | | |
| *3 : Nikkol BB-10 manufactured by Nikko Chemicals Co., Ltd.) | | | | |

### [Test Examples 5 to 7]

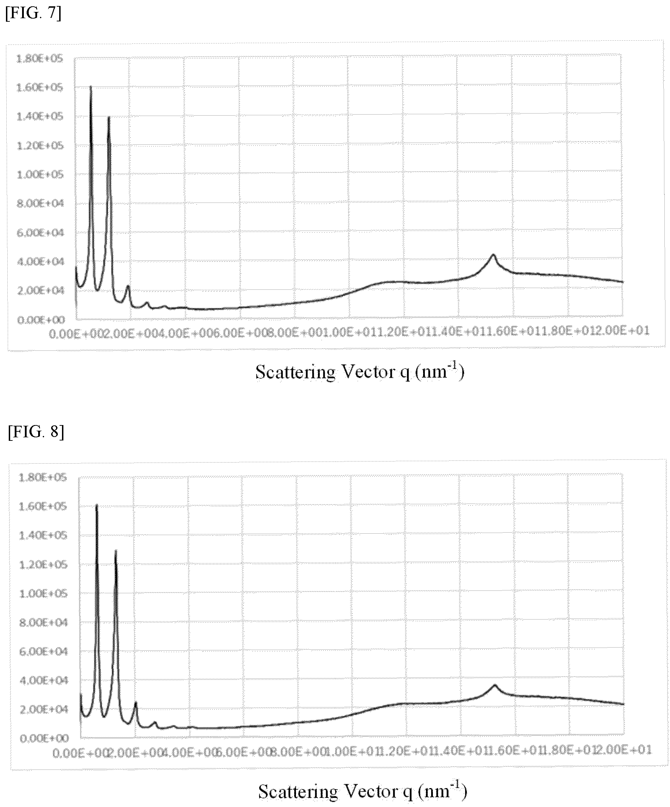

Gel compositions were prepared as in Test Examples 1 to 4, except that the type of ceramide was changed from that in Test Examples 1 to 4. Table 2 shows the compositions. Figures 7 to 9 show small and wide angle X-ray scattering charts of the compositions of Test Examples 5 to 7, respectively.

The presence of a lamellar gel phase having a hexagonal structure was confirmed in all of the compositions. It is therefore believed that the gel composition can be prepared using any of these types of ceramides.

**[Table 2]**

| Test Example | 5 | 6 | 7 |
|---|---|---|---|
| PEG-10 Phytosterol^{*1} | 30.8 | 30.8 | 30.8 |
| PEG-6 Distearate^{*2} | 7.1 | 7.1 | 7.1 |
| Beheneth-10^{*3} | 7.1 | 7.1 | 7.1 |
| Ceramide 2 | 5 | - | - |
| Ceramide 5 | - | 5 | - |
| Ceramide 6 | - | - | 5 |
| Phytosterol | 5 | 5 | 5 |
| Stearic Acid | 5 | 5 | 5 |
| Ion-Exchanged Water | 40 | 40 | 40 |
| Total | 100 | 100 | 100 |

### [Test Examples 8 to 14]

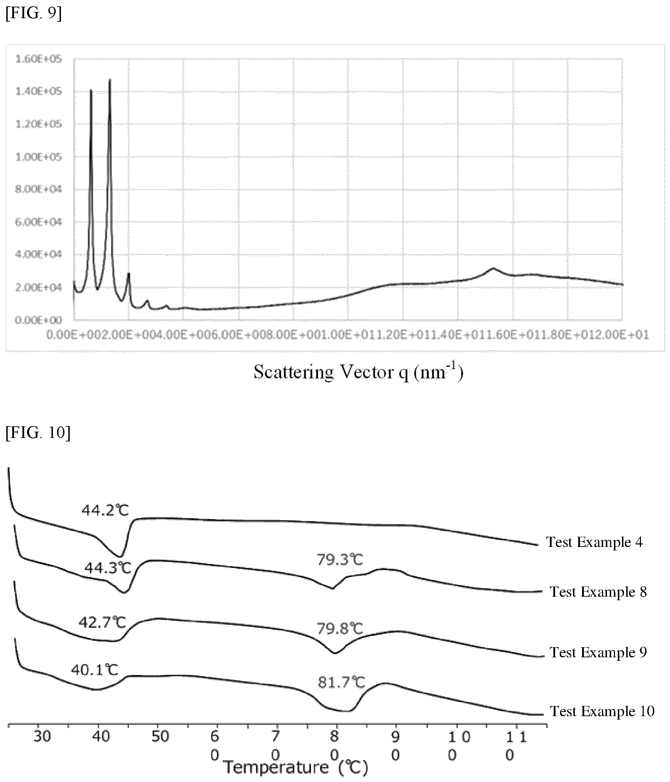

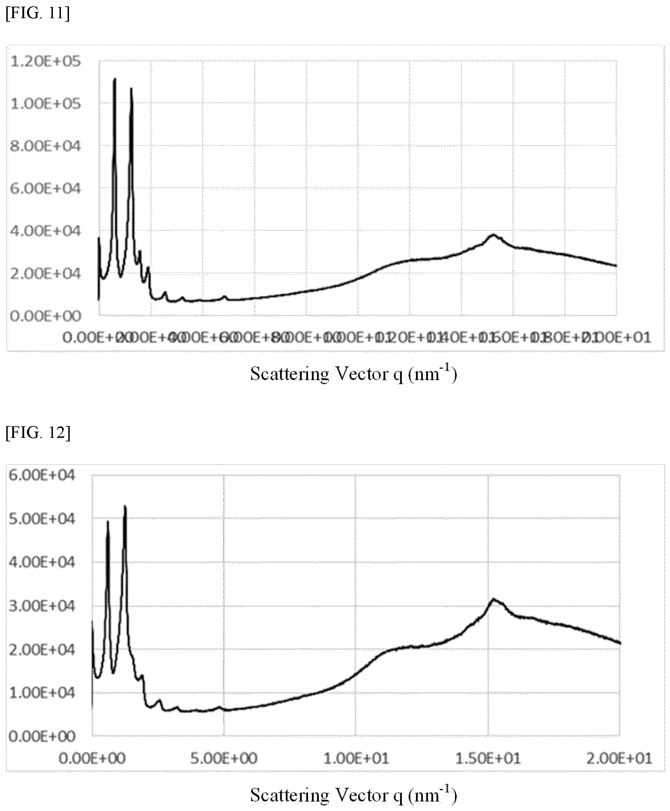

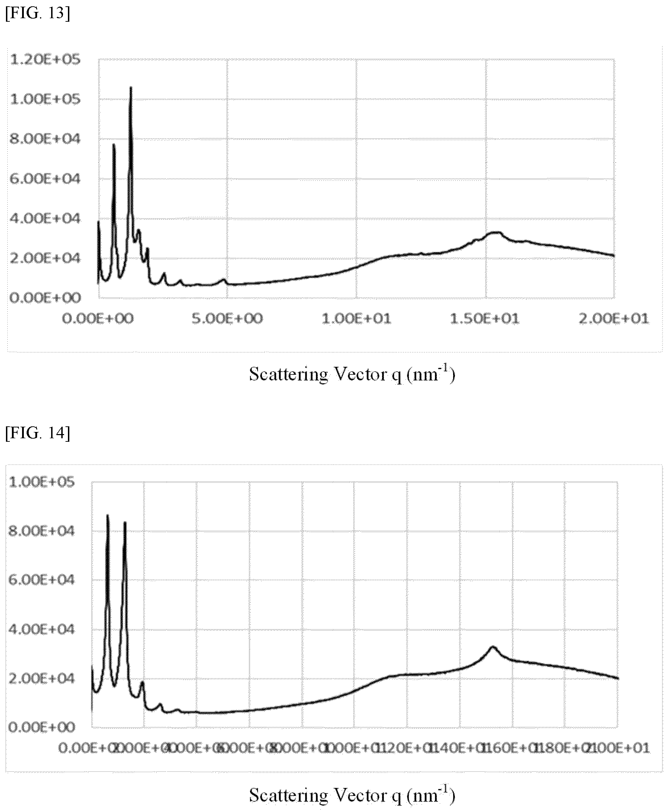

Gel compositions were prepared as in Test Examples 1 to 4, except that the amount of each component blended was changed from the amount in Test Examples 1 to 4. Tables 3 and 4 show the compositions. FIG. 10 shows differential scanning calorimetry charts in Test Examples 4 and 8 to 10. Figures 11 to 14 show small and wide angle X-ray scattering charts of the compositions of Test Examples 11 to 14, respectively.

The compositions of Test Examples 8 to 10, unlike the composition of Test Example 4, were confirmed to show peaks due to a eutectic point at around 80°C. When the composition has a eutectic point, the composition can suppress the deposition of a portion of the crystals.

The presence of a lamellar gel phase having a hexagonal structure was also confirmed in the compositions in Test Examples 11 to 14.

**[Table 3]**

| Test Example | 8 | 9 | 10 |
|---|---|---|---|
| PEG-10 Phytosterol^{*1} | 29.8 | 28.8 | 27.8 |
| PEG-6 Distearate^{*2} | 6.1 | 5.1 | 4.1 |
| Beheneth-10^{*3} | 6.1 | 5.1 | 4.1 |
| Ceramide 3 | 6 | 7 | 8 |
| Phytosterol | 6 | 7 | 8 |
| Stearic Acid | 6 | 7 | 8 |
| Ion-Exchanged Water | 40 | 40 | 40 |
| Total | 100 | 100 | 100 |

**[Table 4]**

| Test Example | 11 | 12 | 13 | 14 |
|---|---|---|---|---|
| PEG-10 Phytosterol^{*1} | 33.3 | 30.8 | 33.3 | 30.8 |
| PEG-6 Distearate^{*2} | 7.1 | 7.1 | 7.1 | 9.6 |
| Beheneth-10^{*3} | 7.1 | 9.6 | 9.6 | 7.1 |
| Ceramide 3 | 5 | 5 | 5 | 2.5 |
| Phytosterol | 2.5 | 2.5 | 2.5 | 5 |
| Stearic Acid | 5 | 5 | 2.5 | 5 |
| Ion-Exchanged Water | 40 | 40 | 40 | 40 |
| Total | 100 | 100 | 100 | 100 |

### [Test Examples 15 to 18]

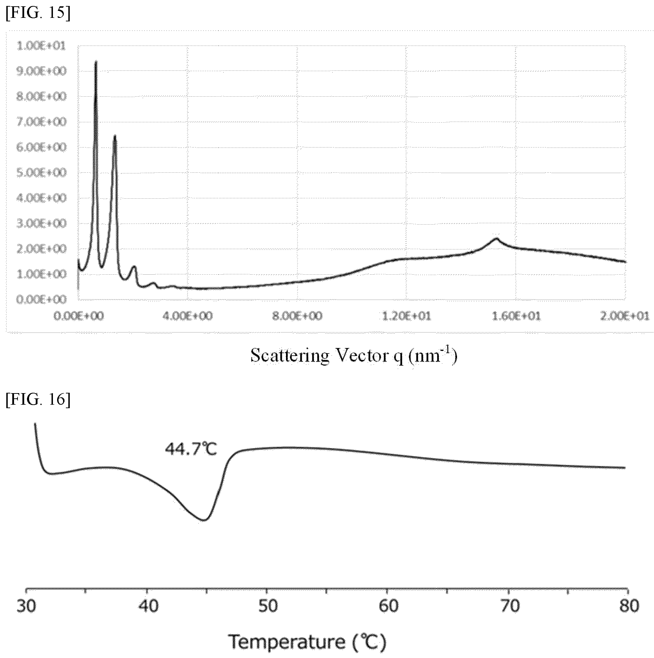

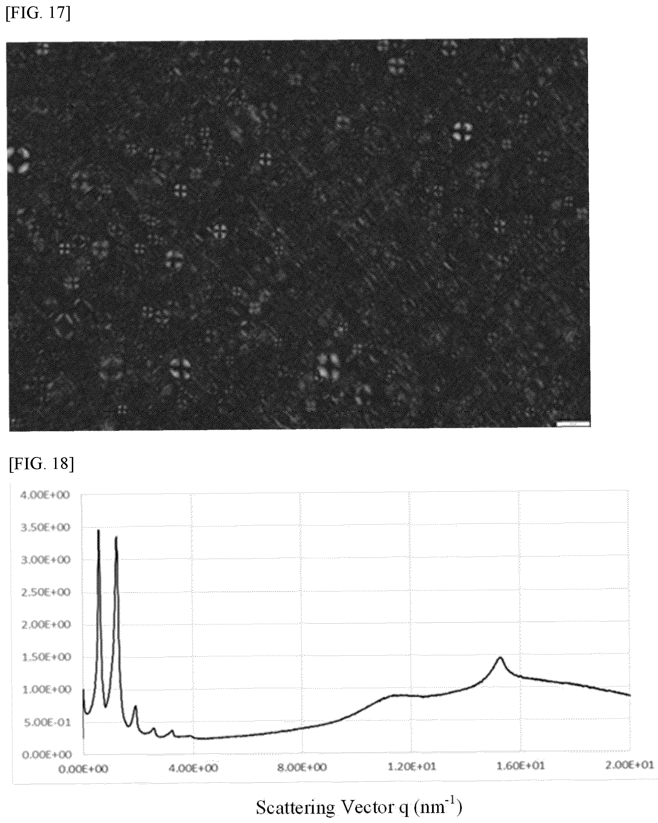

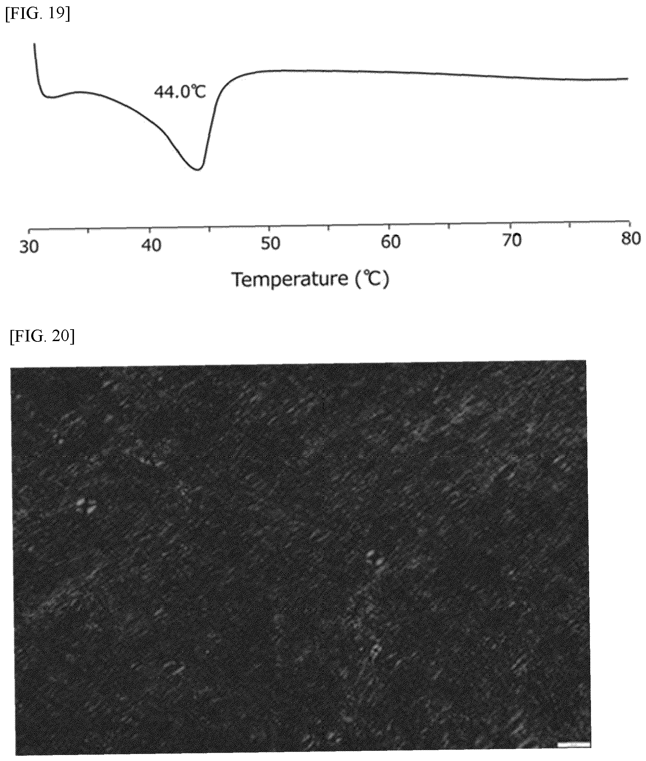

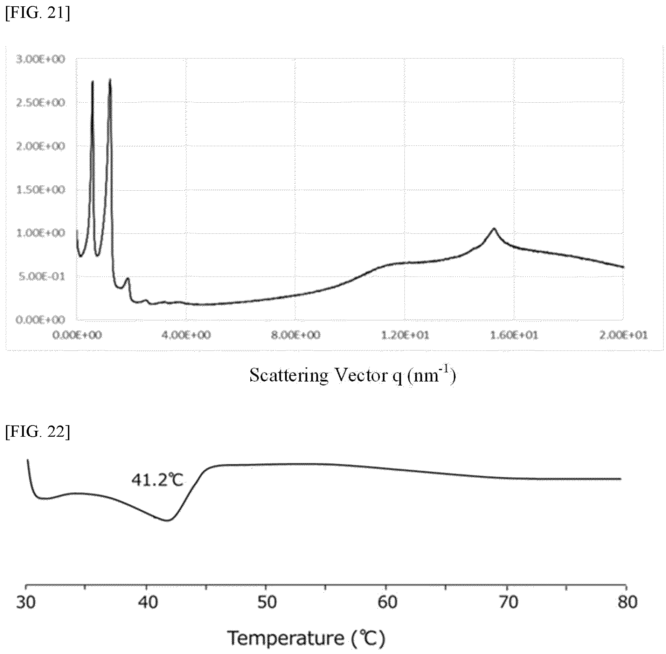

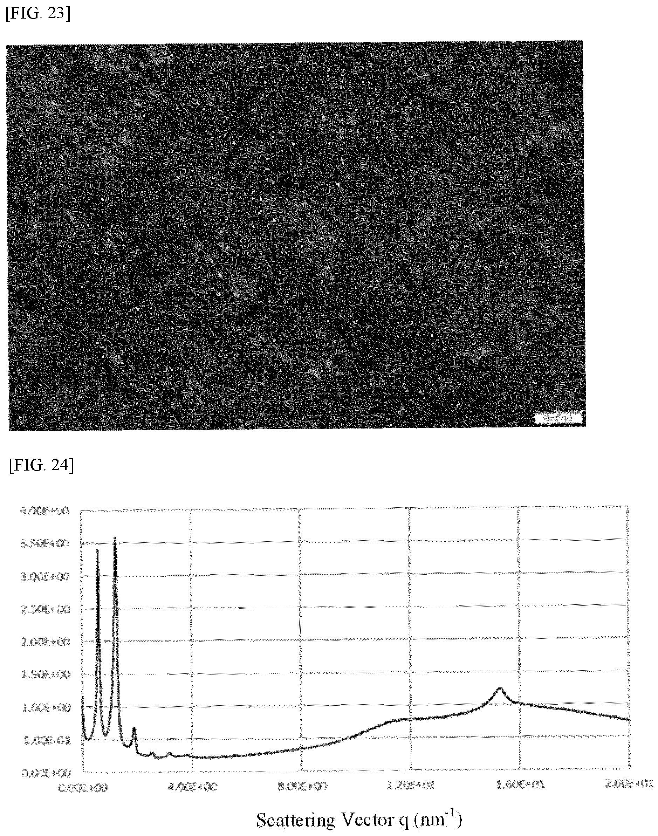

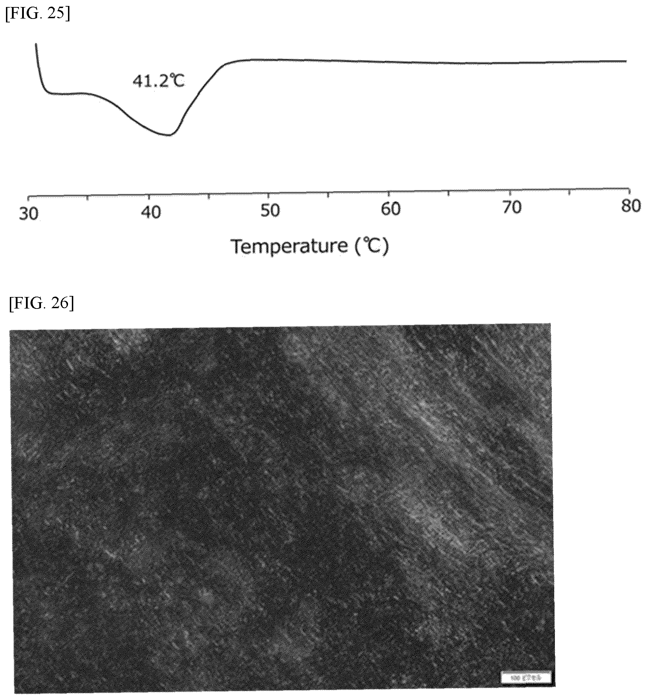

Gel compositions were prepared as in Test Examples 1 to 4, except that the ceramide and the phytosterol in Test Examples 1 to 4 were changed to other compounds. Table 5 shows the compositions. Figures 15, 18, 21, and 24 show small and wide angle X-ray scattering charts of the compositions of Test Examples 15 to 18. Figures 16, 19, 22, and 25 show differential scanning calorimetry charts of the compositions of Test Examples 15 to 18. Figures 17, 20, 23, and 26 show polarizing micrographs of the compositions of Test Examples 15 to 18.

It was confirmed that the gel composition can be prepared by using lecithin instead of ceramide. It was also confirmed that the gel composition can be prepared by using cholesterol and oryzanol instead of phytosterol. It was further confirmed that the compositions of Test Examples 15 to 18 also had a eutectic point as in the test examples described above.

**[Table 5]**

| Test Example | 15 | 16 | 17 | 18 |
|---|---|---|---|---|
| PEG-10 Phytosterol^{*1} | 29.8 | 29.8 | 29.8 | 29.8 |
| PEG-6 Distearate^{*2} | 8.1 | 8.1 | 9.1 | 8.1 |
| Beheneth-10^{*3} | 7.1 | 7.1 | 6.1 | 6.1 |
| Ceramide 3 | 4 | - | 3 | - |
| Soy Lecithin | - | 4 | - | 3 |
| Phytosterol | - | 6 | - | - |
| Cholesterol | 6 | - | - | - |
| γ-Oryzanol | - | - | 6 | 6 |
| Stearic Acid | 5 | 5 | 5 | 5 |
| Ion-Exchanged Water | 40 | 40 | 40 | 40 |
| Total | 100 | 100 | 100 | 100 |

### [Test Example 19]

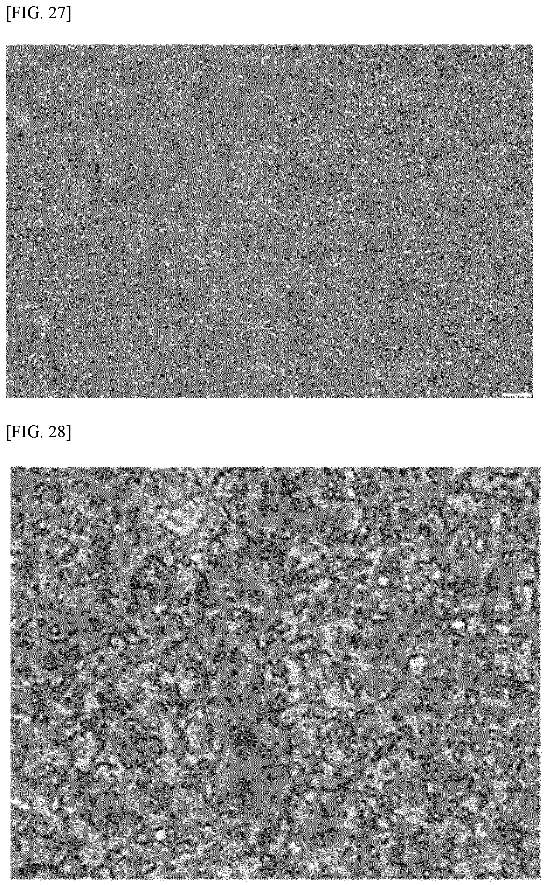

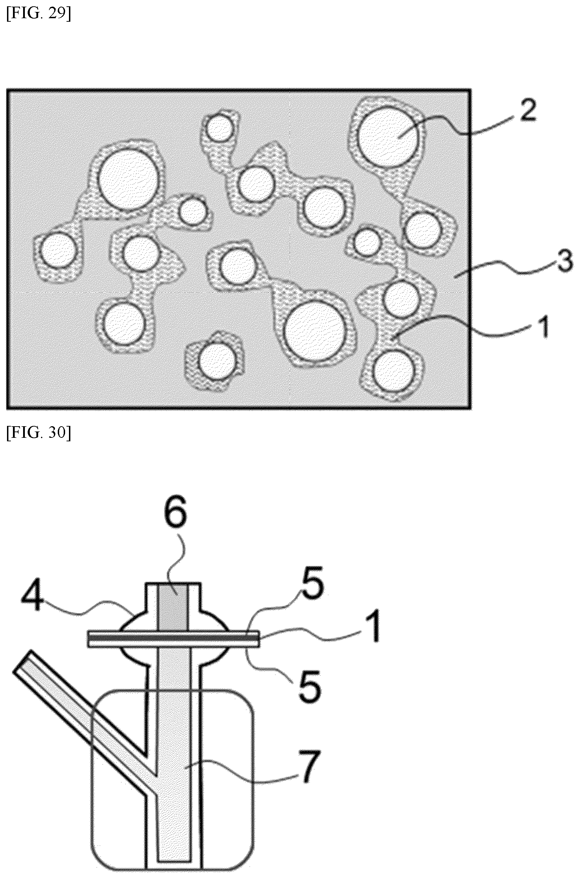

An oil-in-water composition was prepared using a gel composition of the present disclosure. First, PEG-10 phytosterol, PEG-6 distearate, beheneth-10, ceramide 3, phytosterol, and stearic acid were mixed at 80°C, and then a portion of ion-exchanged water at 80°C was added to prepare a gel composition. Next, an oil component at 80°C was added to the gel composition and mixed. Other components were then added and mixed. A polarizing micrograph of the resulting composition was taken. FIG. 27 shows the polarizing micrograph of the composition. FIG. 28 shows a partially enlarged view of the micrograph in FIG. 27. FIG. 29 shows a schematic diagram of an emulsified state of the composition.

It was observed from the micrographs that, as shown in FIG. 29, the oil phase (oil droplets) 2 was incorporated in the gel composition 1 in the aqueous phase 3. This confirmed that the oil-in-water composition stably emulsified by the gel composition can be prepared. The particle diameter of the oil droplets was in the range of 1 to 2 µm. This oil-in-water composition is usable as a skin external composition.

**[Table 6]**

| Test Example | 19 |
|---|---|
| PEG-10 Phytosterol^{*1} | 0.7 |
| PEG-6 Distearate^{*2} | 0.17 |
| Beheneth-10^{*3} | 0.17 |
| Ceramide 3 | 0.1 |
| Phytosterol | 0.1 |
| Stearic Acid | 0.1 |
| Phytosteryl Macadamiate | 1.5 |
| Vaseline | 1.5 |
| Pentaerythrityl Tetraethylhexanoate | 5 |
| Olefin Oligomer | 1.5 |
| Dimethicone | 3 |
| Dipropylene Glycol | 7 |
| Glycerin | 5 |
| 1,3-Butylene Glycol | 5 |
| Ethanol | 5 |
| Carboxyvinyl Polymer | 0.16 |
| Potassium Hydroxide | 0.1 |
| EDTA2Na·2H₂O | 0.03 |
| Succinoglycan | 0.2 |
| Phenoxyethanol | 0.5 |
| Ion-Exchanged Water | Balance |
| Total | 100 |

### [Barrier Function Test]

The barrier function of the gel composition prepared was tested. As a comparative test, a composition having a lamellar gel phase prepared without the addition of ceramide, phytosterol, and a higher fatty acid (Test Example 20) was also tested. FIG. 30 shows a schematic diagram of the test apparatus.

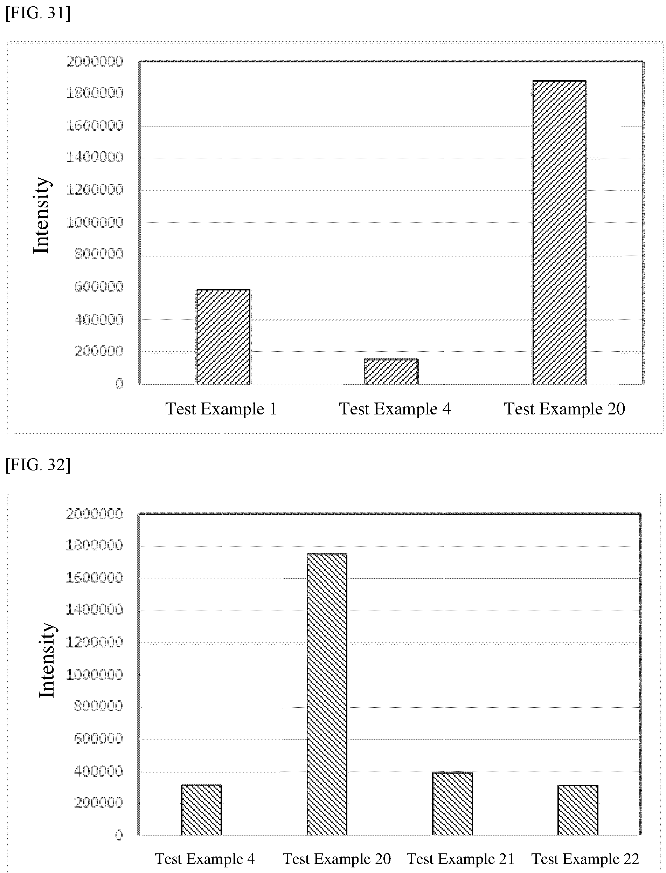

Each of the compositions 1 was applied in an amount of 0.1 g/cm³ to the filter paper 5 and sandwiched with another filter paper 5. This material was dried at room temperature at 50°C overnight. Degassed phosphate buffer was introduced into the receiver 7 side of the Franz diffusion cell 4, and the filter papers 5 sandwiching the composition 1 were set and hydrated for 1 hour. Next, 0.5 mL of the ethanol solution sample 6 of 0.2% fluorescein (manufactured by TCI) was introduced into the donor side, and a 200 µL aliquot of the solution in the receiver 7 was sampled at 23 hours. After the collection, an equal amount of phosphate buffer was added to the receiver 7. The sampled receiver was transferred to a 96-well plate, and fluorescence intensity was measured at 485 nm/535 nm on a microplate reader (ARVO X3, manufactured by PerkinElmer). The measured results are shown in Tables 7 and 8. Each of the measured results shown in Tables 7 and 8 is the average value of three measurements. FIG. 31 shows a comparison of the fluorescence intensities of the gel compositions shown in Table 7. FIG. 32 shows a comparison of the fluorescence intensities of the gel compositions shown in Table 8.

The compositions of Test Examples 1 and 4 exhibited a higher barrier function than that of the composition of Test Example 20, which did not contain ceramide, phytosterol, and a higher fatty acid. The composition of Test Example 4 containing phytosterol and a higher fatty acid exhibited an even higher barrier function.

The Test Example 21 using hydrogenated lecithin instead of ceramide and the Test Example 22 using γ-oryzanol instead of phytosterol also exhibited a high barrier function.

It is therefore believed that the gel composition of the present disclosure when applied to the skin can exhibit a high barrier function.

**[Table 7]**

| Test Example | 1 | 4 | 20 |
|---|---|---|---|
| PEG-10 Phytosterol^{*1} | 35.8 | 30.8 | 35.8 |
| PEG-6 Distearate^{*2} | 7.1 | 7.1 | 12.1 |
| Beheneth-10^{*3} | 12.1 | 7.1 | 12.1 |
| Ceramide 3 | 5 | 5 | - |
| Phytosterol | - | 5 | - |
| Stearic Acid | - | 5 | - |
| Ion-Exchanged Water | 40 | 40 | 40 |
| Total | 100 | 100 | 100 |
| Average Value (n = 3) of Measured Intensities | 583981 | 154454 | 1878877 |

**[Table 8]**

| Test Example | 4 | 20 | 21 | 22 |
|---|---|---|---|---|
| PEG-10 Phytosterol^{*1} | 30.8 | 35.8 | 29.8 | 29.8 |
| PEG-6 Distearate^{*2} | 7.1 | 12.1 | 9.1 | 9.1 |
| Beheneth-10^{*3} | 7.1 | 12.1 | 6.1 | 6.1 |
| Ceramide 3 | 5 | - | - | 3 |
| Hydrogenated Lecithin | - | - | 3 | - |
| Phytosterol | 5 | - | - | - |
| γ-Oryzanol | - | - | 6 | 6 |
| Stearic Acid | 5 | - | 6 | 6 |
| Ion-Exchanged Water | 40 | 40 | 40 | 40 |
| Total | 100 | 100 | 100 | 100 |
| Average Value (n = 3) of Measured Intensities | 315049 | 1751041 | 389968 | 311449 |

While the gel composition and the oil-in-water composition of the present invention have been described based on the embodiments and the examples, the gel composition and the oil-in-water composition are not limited to the embodiments and the examples, and may encompass various modifications, changes, and improvements made to the disclosed elements (including the elements disclosed in the claims, the specification, and the drawings) within the scope of the present invention and based on the fundamental technical idea of the present invention. Furthermore, various combinations, substitutions, or selections of the disclosed elements are possible within the scope of the claims of the present invention.

Further problems, objects, and forms (including changes) of the present invention will become apparent from the entire disclosure of the present invention, including the claims.

The numerical ranges mentioned herein should be interpreted as specifically disclosing herein any numerical values or ranges included within these ranges, even if not otherwise stated.

In the claims in the scope of the claims and the following additional items, any combinations and dependencies are possible.

Some or all of the embodiments may be described as in the following additional items, but are not limited to the following. The additional items can also be combined with the claims set forth in the scope of the claims.

### [Additional Item 1]

A method for producing an oil-in-water composition comprising:
a mixing step of melting a first compound represented by the formula shown in the chemical formula 1, a second compound represented by the formula shown in the chemical formula 2, a third compound represented by the formula shown in the chemical formula 3, and a fourth compound containing at least one selected from the group consisting of ceramide and lecithin to prepare a mixture;
a water addition step of adding water to the mixture to form a lamellar gel phase; and
an incorporation step of incorporating an oil component in the lamellar gel phase.

### [Additional Item 2]

The method according to the additional item, wherein, in the mixing step, a fifth compound containing at least one selected from the group consisting of phytosterol, cholesterol, and oryzanol is further added.

### [Additional Item 3]

The method according to the additional item(s), wherein, in the mixing step, a sixth compound containing at least one selected from the group consisting of higher fatty acids having 14 to 22 carbon atoms is further added.

### [Additional Item 4]

The method according to the additional item(s), wherein, in the water addition step, before addition, the water is heated to a temperature within ±15°C of a heating temperature of the mixture.

### [Additional Item 5]

A method of use comprising applying the gel composition and/or the oil-in-water composition of the present disclosure to a skin external preparation.

### [Industrial Applicability]

The gel composition and the oil-in-water composition of the present disclosure can be applied to the body such as the skin or hair, for example. For example, the oil-in-water composition of the present disclosure can be applied to cosmetics (e.g., foundations, sunscreen cosmetics, makeup bases, and BB creams), hair styling products, cleansing products (e.g., cleansers and shampoos), and the like.

### [Reference Signs List]

- 1: Gel composition
- 2: Oil phase
- 3: Aqueous phase
- 4: Franz diffusion cell
- 5: Filter paper
- 6: Sample
- 7: Receiver

## Claims

1. A gel composition comprising:
a first compound represented by the formula shown in the following chemical formula 1;
a second compound represented by the formula shown in the following chemical formula 2;
a third compound represented by the formula shown in the following chemical formula 3; and
a fourth compound that is a two-chain amphiphile having a nitrogen atom,
wherein the first compound is present in an amount of 49 to 88% by mass, the second compound is present in an amount of 6 to 23% by mass, and the third compound is present in an amount of 6 to 28% by mass, based on a total mass of the first compound, the second compound, and the third compound, and
wherein the fourth compound is present in an amount of 0.04 to 0.22 parts by mass based on 1 part by mass of the total mass: 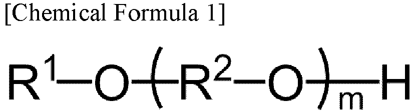
wherein R¹ is at least one selected from the group consisting of a steroid skeleton and a cholesterol skeleton, R² is an alkylene group having 2 to 4 carbon atoms, and m represents an integer of 5 to 30; 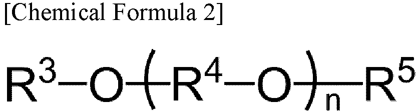
wherein R³ is a straight-chain acyl group or a straight-chain alkyl group having 16 to 24 carbon atoms, R⁴ is an alkylene group having 2 to 4 carbon atoms, R⁵ is a straight-chain acyl group or a straight-chain alkyl group having 16 to 24 carbon atoms, and n represents an integer of 4 to 8; 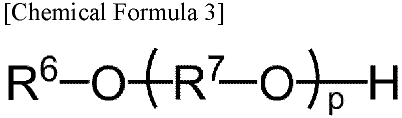
wherein R⁶ is a straight-chain acyl group or a straight-chain alkyl group having 16 to 24 carbon atoms, R⁷ is an alkylene group having 2 to 4 carbon atoms, and p represents an integer of 5 to 30.

2. The gel composition according to claim 1, wherein the first compound, the second compound, the third compound, and the fourth compound form a lamellar gel phase.

3. The gel composition according to claim 1, further comprising a fifth compound having a steroid skeleton,
wherein the fifth compound is present in an amount of 0.04 to 0.22 parts by mass, based on 1 part by mass of the total mass.

4. The gel composition according to claim 3, wherein the first compound, the second compound, the third compound, the fourth compound, and the fifth compound form a lamellar gel phase.

5. The gel composition according to claim 1, further comprising a sixth compound containing at least one selected from the group consisting of higher fatty acids having 14 to 22 carbon atoms,
wherein the sixth compound is present in an amount of 0.04 to 0.22 parts by mass, based on 1 part by mass of the total mass.

6. The gel composition according to claim 5, wherein the first compound, the second compound, the third compound, the fourth compound, and the sixth compound form a lamellar gel phase.

7. The gel composition according to claim 1, further comprising:
a fifth compound having a steroid skeleton; and
a sixth compound containing at least one selected from the group consisting of higher fatty acids having 14 to 22 carbon atoms,
wherein the fifth compound is present in an amount of 0.04 to 0.22 parts by mass based on 1 part by mass of the total mass, and
wherein the sixth compound is present in an amount of 0.04 to 0.22 parts by mass, based on 1 part by mass of the total mass.

8. The gel composition according to claim 7, wherein the first compound, the second compound, the third compound, the fourth compound, the fifth compound, and the sixth compound form a lamellar gel phase.

9. The gel composition according to any one of claims 3, 4, 7, and 8, wherein the fifth compound is at least one selected from the group consisting of phytosterol, cholesterol, and oryzanol.

10. The gel composition according to any one of claims 1 to 9, wherein the total mass constitutes 29 to 66% by mass of the mass of the gel composition.

11. The gel composition according to any one of claims 1 to 10, wherein the fourth compound is at least one selected from the group consisting of ceramide and lecithin.

12. The gel composition according to claim 11, wherein the ceramide is at least one selected from the group consisting of ceramide 1, ceramide 2, ceramide 3, ceramide 3B, ceramide 5, and ceramide 6.

13. The gel composition according to any one of claims 1 to 12, wherein the gel composition has a peak indicating a hexagonal structure in an X-ray scattering pattern.

14. The gel composition according to any one of claims 1 to 13, further comprising water.

15. An oil-in-water composition comprising:
the gel composition according to any one of claims 1 to 14; and
an oil component,
wherein at least a portion of the oil component is incorporated in the gel composition.

16. The oil-in-water composition according to claim 15, further comprising water.

17. The oil-in-water composition according to claim 15 or 16, wherein the oil component is present in an amount of 1 to 50 parts by mass, based on 1 part by mass of the gel composition.

18. The oil-in-water composition according to any one of claims 15 to 17, wherein the oil component contains at least one selected from the group consisting of phytosteryl/octyldodecyl lauroyl glutamate, polybutylene glycol, castor oil, and phytosteryl macadamiate.
